(19) Europäisches Patentamt
European Patent Office
Office européen des brevets

(11) Veröffentlichungsnummer: **0 154 839 B1**

(12) **EUROPÄISCHE PATENTSCHRIFT**

(45) Veröffentlichungstag der Patentschrift: **23.10.91**

(51) Int. Cl.$^5$: **G01N 33/52**

(21) Anmeldenummer: **85101727.7**

(22) Anmeldetag: **16.02.85**

Die Akte enthält technische Angaben, die nach dem Eingang der Anmeldung eingereicht wurden und die nicht in dieser Patentschrift enthalten sind.

# (54) Testvorrichtung und Methode zum Nachweis einer Komponente einer flüssigen Probe.

(30) Priorität: **29.02.84 DE 3407359**

(43) Veröffentlichungstag der Anmeldung:
**18.09.85 Patentblatt 85/38**

(45) Bekanntmachung des Hinweises auf die Patenterteilung:
**23.10.91 Patentblatt 91/43**

(84) Benannte Vertragsstaaten:
**AT BE CH DE FR GB IT LI NL SE**

(56) Entgegenhaltungen:
**EP-A- 0 064 710**
**EP-A- 0 071 169**
**DE-A- 2 602 975**
**US-A- 3 607 093**

(73) Patentinhaber: **BAYER AG**
**W-5090 Leverkusen 1 Bayerwerk(DE)**

(72) Erfinder: **Hildenbrand, Karlheinz, Dr.**
**Bodelschwinghstr. 12**
**W-4150 Krefeld(DE)**
Erfinder: **von Döhren, Hans-Hagen, Dr.**
**Im Muehlenkamp 1**
**W-4630 Bochum-Langendreer(DE)**
Erfinder: **Perrey, Hermann, Dr.**
**Auf der Rheinaue 8**
**W-4150 Krefeld(DE)**
Erfinder: **Frank, Georg, Dr.**
**c/o AMES QSD Miles Laboratories, P.O. Box 70**
**Elkhart, IN 46514(US)**
Erfinder: **Dhein, Rudolf, Dr.**
**Deswatinesstr. 30**
**W-4150 Krefeld(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft ein verbessertes analytisches Element für die spektrophotometrische Analyse einer Komponente in einem wässrigen Testmedium insbesondere einer Körperflüssigkeit Kennzeichnend für das erfindungsgemäße analytische Element ist, daß es mindestens eine asymmetrische, nach dem Koagulationsverfahren hergestellte Polymermembran aufweist, wobei sich die Poren zur Seite der Probenaufgabe hin verengen und das Polymer ausgewählt ist aus der Gruppe Polyamide, Polyamide mit Disulfimidgruppen, Polyethercarbonate, Polyacrylnitril und Polyurethane und daß die Trägerschicht makroskopisch glatt und vorzugsweise unter den Testbedingungen für die Probe undurchlässig ist und die Polymermembran enzymhaltige Reagenzien für den Nachweis der zu bestimmenden Komponenten enthält. Die Bestimmung einer Komponente einer Flüssigkeit mittels "trockener" Reagenzien (z.B. Teststreifen) gewinnt insbesondere in der klinischen Diagnostik immer mehr an Bedeutung. So wird der Nachweis bestimmter Harn-, Serum- oder Blutkomponenten wie Glucose, Bilirubin, Harnstoff oder Proteine in zunehmendem Maße mit Hilfe von Teststreifen durchgeführt. Derartige Analysen sind im Vergleich zu den konventionellen naßchemischen Methoden schneller, einfacher und preisgünstiger.

Bei den üblicherweise verwendeten Teststreifen für flüssige Proben sind die für die Bestimmung des gesuchten Analyten erforderlichen Reagenzien in einem geeigneten, flüssigkeitsaufnehmenden Trägermaterial enthalten. Wird die flüssige Probe auf diesen Träger aufgegeben, so kommt es zur Diffusion der Flüssigkeit ins Trägerinnere (Reaktionsraum), wobei die Nachweisreagenzien mit der zu analysierenden Probenkomponente z.B. eine spezifische, konzentrationsabhängige Färbung ergeben.

Als flüssigkeitsaufnehmende Trägermaterialien wurden anfänglich einfache und in der Folgezeit chemisch modifizierte Papiere eingesetzt, die mit den Nachweisreagenzien getränkt wurden. Wegen ihrer mangelnden Homogenität bezüglich Schichtdicke und Zusammensetzung sind jedoch Papiere für quantitative Bestimmungen wenig geeignet.

Einen wichtigen Fortschritt in der quantitativen Test-Streifen-Diagnostik stellte die Verwendung von polymerem Trägermaterial mittels geeigneter Beschichtungstechnologien dar.

So wird in der DE-AS 2 332 760 eine mehrschichtige Testvorrichtung aus transparentem Träger, Gelatineschicht und mikroporöser, füllstoffhaltiger Celluloseacetatschicht beschrieben. Die Gelatineschicht enthält die Reagenzien und dient damit als Reaktions- und Nachweiszone. Funktion der mikroporösen Celluloseacetatschicht ist die homogene Verteilung der Probe, die Abtrennung der Erythrocyten und die Reflexion der von der Trägerseite eingestrahlten Meßstrahlung.

Ein Vorteil von Gelatineschichten enthaltenden Testvorrichtungen ist, daß die zu ihrer Herstellung benutzte Beschichtungstechnologie aus der Fotographie bekannt und technisch ausgereift ist. Nachteilig ist bei solchen Systemen wie auch bei anderen Testvorrichtungen, die in Wasser quellende Polymere (z.B. Agarose) enthalten, daß neben der Nachweisreaktion auch noch Quellvorgänge ablaufen, so daß es erst nach längerer Zeit zum Stillstand der Reaktion kommt. Man kann somit zur schnellen Analyse nicht die Endpunktbestimmung sondern nur die Kinetik der Reaktion heranziehen. Um quantitative Analysen zu ermöglichen, muß man die Probe dosieren. Gelatine ist darüberhinaus nicht ideal wegen der begrenzten Haltbarkeit der biochemischen Nachweisreagentien in Gelatine und wegen der begrenzten Stabilität der bei der Nachweisreaktion gebildeten Färbung (eine Auswertung der Analyse erst nach mehreren Tagen ist daher nicht möglich). Außerdem ist Gelatine anfällig gegenüber Proteasen, die als Verunreinigung in den für die Nachweisreaktion benötigten Enzymen üblicherweise vorkommen.

Ein weiteres Teststreifensystem auf Polymerbasis zur Bestimmung von Inhaltsstoffen in Körperflüssigkeiten wird in der Europ. Patentanmeldung 0 064 710 beschrieben. Als Matrix für die Reagenzien dient hierbei ein poröser Polymerfilm, hergestellt durch Eintrocknen eines Latex (z.B. einer wäßrigen Polyvinylpropionat-Dispersion) in Gegenwart eines Porenbildners (z.B. Kieselgel). Die zu analysierende, flüssige Probe wird direkt auf die Reagenzschicht, die sich auf einer PVC-Folie befindet, aufgegeben. Nach einer bestimmten Zeit werden der Probenüberschuß bzw. die Erythrozyten abgewischt und die Farbreaktion von der trägerabgewandten Seite aus beobachtet bzw. reflektometrisch bestimmt.

Unbefriedigend bei diesem System (ganz allgemein_ bei Systemen, bei denen die Probe direkt auf die Reagenzschicht aufgebracht wird) ist das sogenannte Ausbluten. Es können sich nämlich Nachweisreagenzien aus der Reagenz-Schicht, insbesondere wasserlösliche (z.B. Enzyme), im Probenüberschuß lösen, die dann beim Abwischen dem System entzogen werden und zu einer Verfälschung der Ergebnisse führen. Aus diesem Grunde sind auch Teststreifenentwicklungen interessant, bei denen die Auswertung über einen transparenten Träger möglich ist, so daß man auf das Abwischen der Probe verzichten kann.

Weitere Nachteile des Systems von EP-A 64 710 sind, daß man die Probe relativ lange (z.B. 2 Min.) einwirken lassen muß und daß die Einstellung gezielter, insbesondere relativ großer Poren (im Bereich von µm) problematisch ist. Derartige großporige Systeme wären vor allem für den Nachweis von höhermolekularen Analyten interessant.

In EP 71 169 werden offenporig-mikroporös ausgebildete Formkörper mit inhärenter latenter Strukturumwandelbarkeit beschrieben, d.h. die Formkörper sind "latent transparent". Diese Formkörper können für analytische und diagnostische Verfahren auf immunologischer Basis verwendet werden. Nachteilig ist, daß die Strukturumwandlung nur dann vertretbar schnell erfolgt, wenn die Formkörper auf eine Temperatur zwischen 50 und 220 °C erhitzt werden und/oder bei chemischer Einwirkung wie Aceton gegebenenfalls noch kombiniert mit Krafteinwirkung. Damit sind die Formkörper nur unter solchen Bedingungen in der Analytik einsetzbar, da sonst die langsame Strukturumwandlung die Analysenreaktion überlagert und damit stört. Enzyme die man vielfach in der Diagnostik verwendet, werden unter solchen Bedingungen denaturiert und damit zerstört.

Ziel der vorliegenden Erfindung ist die Entwicklung eines neuen Testmittels, insbesondere für Komponenten im Vollblut, das bei einfacher Handhabung möglichst quantitative Ergebnisse liefert. Eine einfache Handhabung ist insofern wichtig, als die Teststreifendiagnostik in zunehmendem Maße auch von Nicht-Fachleuten angewandt wird, für die insbesondere eine genaue Dosierung der Probenmenge oder auch das Abwischen des Probenüberschusses nach definierten Zeiten problematisch ist.

Das Testmittel soll einfach und in gleichbleibender Qualitat herstellbar sein und insbesondere folgende Eigenschaften besitzen:

- reproduzierbare Abhängigkeit der Farbreaktion von der Analyt -Konzentration;
- intensive Färbungen;
- schnelle Reaktion (Endpunktbestimmung);
- Ablesbarkeit von der Trägerseite und (nach Abwischen) von der Aufgabenseite aus;
- Messung von Vollblut möglich;
- gute Haltbarkeit der Farben und des gesamten Systems;
- einfache Einstellung gezielter Porengrößen;
- Unabhängigkeit der Analysenwerte vom aufgegebenen Probenvolumen;
- nicht quellende Polymermatrix;
- kein Ausbluten.

Es wurde nun überraschenderweise gefunden, daß nach der Methode der Membranherstellung durch Koagulation von Polymerlösungen Testvorrichtungen mit Polymerschichten für die Probenapplikation hergestellt werden können, die alleine oder auch in Kombination mit weiteren Elementen allen Anforderungen der klinischen Diagnostik genügen, ohne die oben genannten Mängel aufzuweisen. So eignet sich zur Herstellung von Membranen nach der Koagulationsmethode eine Vielzahl von Polymersystemen mit unterschiedlichster chemischer Konstitution (z.B. hydrophil; hydrophob; saure bzw. basische Ionenaustauscherfunktionen), die spezifische Trennungen ermöglichen und biologisch nicht abbaubar sind. Außerdem lassen sich über das Herstellverfahren Membranen mit unterschiedlichen definierten Porengrößen und Porenvolumina herstellen, wodurch die gezielte Separation von störenden Bestandteilen ermöglicht wird und das System selbstdosierend wirkt.

Gegenstand der vorliegenden Erfindung ist eine Testvorrichtung für den Nachweis einer Komponente in einer flüssigen Probe, insbesondere in einer Körperflüssigkeit wie Blut oder Urin, wobei die Vorrichtung eine Trägerschicht, eine mikroporöse Polymerschicht, gegebenenfalls weitere Schichten, sowie in einer oder mehreren der Schichten inkorporiert enzymhaltige Reagentien für den Nachweis der zu bestimmenden Komponenten umfaßt. Kennzeichnend für die Erfindung ist einerseits die Verwendung einer mikroporösen Polymerschicht, welche eine nach der Koagulationsmethode hergestellte Membran ist, die eine asymmetrische Porenstruktur aufweist, wobei sich die Poren zu der für die Probenaufgabe vorgesehenen Seite der Testvorrichtung hin verengen, und andererseits die Verwendung einer makroskopisch glatten Trägerschicht, die vorzugsweise unter den Testbedingungen für die Probe undurchlässig ist.

Erfindungsgemäß kommen mikroporöse Filme aus vollsynthetischen Polymeren in Frage, die mit definierten Porenvolumina und variablen Porengrößen hergestellt werden können. Der vollsynthetische Charakter der Polymeren ist deswegen wichtig, da diese - im Gegensatz zu natürlichen oder halbsynthetischen Produkten - in hoher Reproduzierbarkeit hergestellt werden können und eine einfache Qualitätskontrolle ermöglichen.

Mikroporöse Polymerfilme (Membranen), die unter Anwendung eines äußeren Druckes zur großtechnischen Trennung von molekularen Mischungen eingesetzt werden, sind schon seit längerem bekannt und auch kommerziell erhältlich. Es gibt mehrere Verfahren, derartige Membranen herzustellen, wobei die sogenannte "Phaseninversions-Methode" die größte Bedeutung erreicht hat. Über die Grundlagen dieser Technologie informiert z.B. H. Strathmann, "Trennungen von molekularen Mischungen mit Hilfe synthetischer Membranen", Steinkopfverlag Darmstadt (1979).

Innerhalb des Phaseninversionsverfahrens gibt es verschiedene Varianten. Beim Koagulationsverfahren

wird im Prinzip so vorgegangen, daß man einen festen Träger mit einer Polymerlösung (Gießlösung) von gleichmäßiger Dicke (z.B. 100 μm) beschichtet, gegebenenfalls einer Atmosphäre, welche ein Nichtlösungsmittel (bevorzugt Wasser) für das Polymere in Dampfform enthält, bis zur teilweisen oder vollständigen Gelierung der Lösung aussetzt, und anschließend in ein Koagulationsbad taucht, wobei ein fester, mikroporöser Film mit asymmetrischer Struktur entsteht. Bleibt der Membranfilm während der Koagulation auf dem festen Träger haften (bei Verwendung von speziellen porösen Polymervliesen oder Polymerfolien), so erhält man trägergestützte Membranen. Löst sich der Membranfilm während der Koagulation vom festen Träger (z.B. bei Verwendung von Glas), so entstehen trägerfreie Membranen. Die Koagulationsflüssigkeit ist so beschaffen, daß sie mit dem Lösungsmittel der Gießlösung mischbar, für das Polymere aber ein Fällmittel ist.

Typisch für diese Variante ist, daß die Koagulation (Porenbildung) im wesentlichen erst beim Eintauchen in die Koagulationsflüssigkeit erfolgt und daß asymmetrische Membranstrukturen entstehen. Asymmetrisch bedeutet dabei, daß sich die Poren - von der Membranunterseite (Trägerseite) aus betrachtet - zur Membranoberfläche hin verengen.

Das Verhältnis der durchschnittlichen Porendurchmesser an der Membranunterseite zu jener an der Membranoberfläche (bestimmbar z.B. mittels elektronenmikroskopischer Aufnahmen) ist dabei in der Regel größer als 5 : 1, bevorzugt größer als 10 : 1, besonders bevorzugt größer als 30 : 1. Wie sich zeigte, hat die erfindungsgemäße Verwendung derartiger Membranen den Vorteil, daß bei Produktaufgabe auf die Membranoberfläche eine Verstopfung der Poren verhindert wird.

Die Mehrzahl der käuflichen Membranen wird nach diesem Verfahren hergestellt. Es wurde bereits vorgeschlagen, derartige Membranen direkt als Trägermatrix für Nachweissysteme einzusetzen (US 3 607 093), indem die fertigen Membranen nachträglich mit den Testreagenzien getränkt wurden. Die käuflichen, trägergestützten Membranen erfüllen jedoch die in der Praxis an die Reproduzierbarkeit gestellten Anforderungen nicht.

Diese Membranen befinden sich nämlich auf porösen Trägervliesen, z.B. aus Polyethylen, Polypropylen oder Polyester, die makroskopisch nicht glatt sind. Dadurch ist die Membranschichtdicke und damit das Porenvolumen relativ starken Schwankungen unterworfen, was zu Streuungen bei der Nachweisreaktion führt. Außerdem hat der poröse, flüssigkeitsaufnehmende Charakter der Trägervliese zur Folge, daß nicht allein das Porenvolumen der Membran für die aufgenommene Flüssigkeitsmenge verantwortlich ist, sondern auch der poröse Träger, der aufgrund von Kapillarkräften Flüssigkeit aufsaugt.

Trägerfreie asymmetrische Membranen wurden bisher noch nicht für die Herstellung von Testvorrichtungen eingesetzt oder vorgeschlagen. Sie sind jedoch erfindungsgemäß weniger bevorzugt, da sie schwierig zu handhaben sind und in der Regel nur dann getrocknet werden können, wenn sie mit Konservierungsmitteln imprägniert sind. Sie erfordern daher bei der Herstellung eines Testmittels einen weiteren Arbeitsschritt, nämlich das Aufbringen auf einen Träger. Hierzu können Klebstoffe verwendet werden, die jedoch zu weiteren Komplikationen führen können (z.B. Anlösen der Membran; Flüssigkeitsaufnahme).

Das Einbringen der Nachweisreagentien in die fertigen Membranen erfolgt bei dem im US-Patent 3 607 093 beschriebenen System durch Tränken. Da in der Regel sowohl organische als auch wasserlösliche Reagenzien eingebrach werden müssen, ist man sowohl auf Tränkung in organischer Lösung als auch auf eine Tränkung in wäßriger Lösung angewiesen.

Man ist daher auf Membranen beschränkt, die gegen das entsprechende organische Lösungsmittel beständig sind. Außerdem neigen derartige Systeme, die mit den Nachweisreagenzien lediglich getränkt werden, zum Ausbluten.

Eine weitere Variante der Membranherstellung nach der Phaseninversionsmethode beruht darauf, daß man ein Polymer in einem Gemisch aus einem guten, leicht verdampfbaren und einem schlechten, höher siedenden Lösungsmittel löst. Verstreicht man eine derartige Lösung zu einem Film und führt Wärme zu, so verdampft das gute, leicht siedende Lösungsmittel zuerst, während sich das für das Polymer schlechte Lösungsmittel anreichert und das System zur Koagulation bringt. Anschließend kann gegebenenfalls zum Auswaschen der Reste des hochsiedenden Lösungsmittels die Membran noch in ein flüssiges Bad getaucht werden, das jedoch im Gegensatz zu der oben beschriebenen Koagulation im Fällbad nicht wesentlich zur Membranbildung beiträgt.

Nach dieser Methode erhält man keine asymmetrische Strukturen. Außerdem können praktisch nur solche Polymere verwendet werden, für die es ein gutes, leicht siedendes Lösungsmittel gibt, was die Polymerauswahl sehr einschränkt. So wurden nach dieser Methode bisher in der Praxis lediglich die halbsynthetischen Cellulosederivate (z.B. Celluloseacetat, Cellulosenitrat), für die Aceton ein sehr gutes Lösungsmittel ist, zu Membranen verarbeitet. Beispielsweise wurden die gemäß DE-AS 2 332 760 verwendeten, auf Gelatineschichten befindlichen Filterschichten aus Celluloseacetat ("blush-Polymer-Schichten") in dem Lösungsmittelsysten Aceton/Toluol hergestellt.

Diese Methode wurde, wie in der DOS 2 602 975 beschrieben, auch schon zur Herstellung einschichtiger Nachweissysteme verwendet, wobei die Reagenzien in die Polymerlösung eingearbeitet wurden. Nach diesem Verfahren wurden daher direkt poröse, die Nachweisreagenzien enthaltende Membranen erhalten. Die Herstellung der Testelemente gemäß DOS 2 602 975 erfolgt auf einer Glasplatte, von der die Membranen nach dem Trocknen abgezogen und auf eine Kunststoffolie aufgeklebt wurde. Hierbei ergeben sich die oben beschriebenen Schwierigkeiten der trägerfreien Membranen. In DOS 2 602 975 wird auch beschrieben, daß man die Nachweissysteme direkt auf einem integralen Substrat herstellen kann, indem man die Glasplatte durch eine Polymerfolie ersetzt, die so beschaffen sein muß, daß sie mit dem verwendeten Lösungsmittelsystem chemisch oder physikalisch reagiert, wobei es zum Verschmelzen der Membran mit der Trägerfolie kommt. Bei diesem Prozeß ist man jedoch sehr wenig variabel, da die Lösungsmittelzusammensetzung und die Abdampfzeit danach gewählt werden müssen, in welcher Porosität man die Membran herstellen will. Man wird demnach den Träger unterschiedlich stark beeinflussen, je nachdem, ob man fein- oder grobporöse Membranen herstellen will. Bei transparenten Trägern wird durch das Anlösen in der Regel auch die Lichtdurchlässigkeit beeinträchtigt, wodurch das Auswerten von der Trägerseite aus problematisch ist.

Außerdem kommt es bei Polymerfolien, die von Lösungsmitteln angegriffen werden, in der Regel zu irreversiblen Veränderungen, wie Quellen, Schrumpfen oder Unebenheiten, insbesondere wenn die Kontaktzeit zwischen Lösungsmittel und Polymerträger relativ lange dauert, was bei der dort beschriebenen Art der Membranherstellung der Fall ist. Diese Methode ist demnach zur Herstellung von trägergestützten mikroporösen Polymerfilmen, die die Anforderungen für Reagenzienträger erfüllen sollen, nicht geeignet.

Details der Herstellung von mikroporösen Flächengebilden nach dem erfindungsgemäß anzuwendenden Koagulationsverfahren werden in einer Vielzahl von Veröffentlichungen angegeben. So wird in der Deutchen Patentschrift 1 110 607 für die Koagulation von Polyurethanen auf Polyetherbasis vorgeschlagen, hygroskopische Polyurethanlösungen (als Lösungsmittel dient dabei z.B. Dimethylformamid) der Einwirkung einer wasserdampfhaltigen, gegebenenfalls in Zirkulation versetzten Atmosphäre auszusetzen, welche eine relative Feuchtigkeit von 15 bis 100 % bei einer Temperatur des trockenen Thermometers von 10 bis 38° C besitzt. Wegen der Absorption von Wasser infolge der Hygroskopie des Lösungsmittels beginnt das Polyurethan aus der Lösung von der Oberfläche her auszufallen, wahrscheinlich unter Präformierung der mikroporösen Struktur. Beim Einlegen der auf diese Weise vorgelierten Filme oder Überzüge in Wasser wird unter Koagulation der Lösung das hygroskopische Lösungsmittel aus dem Film vollständig entfernt.

Die DE-OS 1 444 163 gibt ein etwas modifiziertes Verfahren an: Durch Zugabe von kleineren Anteilen an Nichtlösungsmitteln (z.B. Wasser) wird die Polyurethanlösung erst in den Zustand beginnender Phasentrennung, d.h. in eine leicht trübe, dispersionsartige Form gebracht, bevor sie (nach dem Aufstreichen in Flächenform) direkt, also ohne Vorgelierung in feuchter Atmosphäre, durch Eintauchen in das Nichtlösungsmittel koaguliert wird.

In der DE-OS 1 444 165 wird ein weiteres Verfahren angegeben, wonach sich die Polymerlösung ohne Vorgelierung durch indirekte Koagulation in einer Mischung aus Nichtlösungsmittel und Lösungsmittel (z.B. Dimethylformamid/$H_2O$ in einem Mischungsverhältnis zwischen 10:90 und 95:5) in mikroporöse Folien überführen läßt.

Nach einer weiteren Variante, welche in der belgischen Patentschrift 624 250 beschrieben ist, setzt man der Polymerlösung so viel Nichtlöser zu, daß sich das Polymer als Gel abscheidet. Man streicht dann erst dieses Gel auf ein Substrat und koaguliert mit Nichtlösungsmittel (Wasser) zu einem mikroporösen Gebilde.

In der DE-AS 1 238 206 wird angegeben, daß eine direkte Koagulation von Elastomerlösungen dann zu mikroporösen Strukturen führt, wenn man die beschichteten Substrate in Bädern, welche bis in die Nähe des Siedepunktes der Badflüssigkeit erhitzt sind, z.B. in heißem Wasser von 95° C, koaguliert.

Verbesserte Ergebnisse werden erhalten, wenn auch die Vorgelierung bei erhöhter Temperatur stattfindet. So beschreibt die DE-OS 2 025 616 ein Verfahren zur Herstellung mikroporöser Flächengebilde, bei dem man eine dünne Schicht einer Polyurethanlösung einer Wasserdampfatmosphäre von mindestens 50 % relativer Feuchtigkeit bei Temperaturen oberhalb von 65° C aussetzt, anschließend in wäßrigen Koagulationsbädern die Hauptmenge des Lösungsmittels entfernt und dann trocknet.

Gemäß DE-OS 2 125 908 wird über eine Schicht einer Polyurethanlösung Wasserdampf mit einer Temperatur zwischen 101° C und 190° C geführt, bis der Gehalt der Schicht an organischem Lösungsmittel auf weniger als 50 Gew.-% gesunken und die Schicht in ein festes, mechanisch stabiles mikroporöses Flächengebilde übergegangen ist. Dieses Verfahren hat insbesondere den Vorteil, daß in kurzer Zeit und in einem einzigen Verfahrensschritt aus einer Polyurethanlösung ein mikroporöses Endprodukt entsteht.

Bei den genannten Verfahren können den Polymerlösungen zwecks Verbesserung der Koagulierbarkeit bestimmte Koagulationshilfsmittel zugesetzt werden. So beschreiben die DE-AS 1 270 276, die DE-OS 1

694 171 und die DE-OS 1 769 277 Verfahren zur Herstellung wasserdampfdurchlässiger Flächengebilde, bei denen Lösungen von 90 bis 70 Gew.-Teilen an Polyurethanen bzw. Polyharnstoffen und 10 bis 30 Gew.-Teilen an hochmolekularen, im wesentlichen linearen, kationischen Polyurethanen, die 0,5 bis 2,0 Gew.-% quartäre Ammonium-Stickstoff-Atome enthalten, gegebenenfalls nach Gelierung an feuchter Luft, mit Wasser bzw. einem Gemisch aus Wasser und Lösungsmittel koaguliert werden. Neben den kationischen Polyurethanen können diese Lösungen als zusätzliche Koagulationsregulatoren auch noch anionische Gerbstoffe enthalten.

In vielen Fällen kann gemäß DE-OS 2 427 274 eine weitere Verbesserung dadurch erreicht werden, daß die zu koagulierenden Polyurethanlösungen bestimmte kationische bzw. anionische Polyurethanharnstoffsuspensionen allein, oder vorzugsweise gleichzeitig kationische und anionische Polyurethan(harnstoff)e in Salzform enthalten. Es gelingt auf diese Weise, auch die Koagulation von schwer verarbeitbaren Polyurethanlösungen so zu regulieren, daß Flächengebilde mit befriedigender Mikroporösität entstehen.

Nach den in den genannten Druckschriften beschriebenen Verfahren lassen sich praktisch aus jedem löslichen Polymeren mikroporöse Membranen herstellen, wobei über verschiedene Parameter (z.B. Konzentration der Gießlösung, Temperatur, Additive, Natur der Koagulationsflüssigkeit) - gegebenenfalls nach wenigen Vorversuchen - gezielte Porengrößen einstellbar sind.

Aus diesen Möglichkeiten ergeben sich auch die Vorteile der vorliegenden Erfindung. So kann die polymere Membran in einfacher Weise bzgl. Zusammensetzung und Porösität auf das beabsichtigte Nachweissystem abgestimmt werden.

In den meisten Fällen ist es erfindungsgemäß bevorzugt, die Koagulation direkt in einem wäßrigen Koagulationsbad, d.h. ohne besondere Vorbehandlung, auszuführen.

Die Eigenschaften der erfindungsgemäßen Nachweiselemente (Homogenität und Intensität der Farbreaktion, Ausbluten, Haltbarkeit des Nachweissystems, Erythrozytenabwischbarkeit und Geschwindigkeit der Nachweisreaktion) hängen stark von der Art des verwendeten Polymersystems ab.

Um die Forderungen nach einfacher maschineller Herstellbarkeit, definiertem Porenvolumen und Auswerten von der Trägerrückseite aus zu erfüllen, werden erfindungsgemäß vorzugsweise solche Polymer-Gießlösungen eingesetzt, daß die Membranherstellung direkt auf einem makroskopisch glatten, undurchlässigen Träger erfolgen kann. Werden die Gießlösungen konventioneller Membranen (z.B. Celluloseacetat oder Polysulfon) auf glatte, undurchlässige Folien aufgetragen, so löst sich der bei der Koagulation entstehende feste Film vom Träger, falls der Träger nicht vom Lösungsmittel so stark angegriffen wird, daß er mit der Membran verschmilzt. Dies führt jedoch zu einer Streuung der Meßergebnisse. Um dieses Problem zu vermeiden, muß bei der direkten Herstellung trägergestützter Membranen das System Gießlösung-Träger so aufeinander abgestimmt werden, daß die Gießlösung eine hohe Affinität zum Träger hat, ohne ihn anzugreifen, d.h. aufzulösen.

Als günstig haben sich solche Polymersysteme erwiesen, die sich in an sich bekannter Weise bezüglich ihrer Hydrophilie/Hydrophobie-Balance variieren lassen. In diesem Zusammenhang sind besonders Polymere mit ionischen Gruppen von Vorteil, die gegebenenfalls auch als Komponente in einer Polymermischung eingesetzt werden können. Die hydrophilen (bzw. hydrophoben) Eigenschaften der Polymermembran können z.B. von Bedeutung für die Optimierung der Farbreaktion sein (häufig wird die Farbe intensiver, wenn das Polymer ionische Gruppen enthält).

Die Polymere, aus denen sich geeignete Gießlösungen für die erfindungsgemäß beanspruchten Nachweiselemente herstellen lassen, sind: Polyamide, Polyamide mit Disulfimidgruppen

$$\begin{array}{c} Na^{\oplus} \\ \ominus \\ (-SO_2-N-SO_2-) \end{array}$$

(siehe z.B. US-PS 4 269 967), Polyethercarbonate (siehe z.B. DE-OS 2 251 066) Polyacrylnitril und Polyurethane, wie sie z.B. in der DE-OS 24 27 274 und den dort zitierten Druckschriften beschrieben werden.

Bevorzugte Gießlösungen werden erhalten, wenn man den Lösungen der genannten Polymeren an sich bekannte wäßrige Polymerdispersionen, die vorzugsweise ionische Gruppen aufweisen, z.B. aus Polyvinylverbindungen, Vinyl-Mischpolymerisaten, Polystyrolsulfonsäuren, Polyamiden oder Polyurethanen, zumischt. Ganz besonders geeignet sind Gießlösungen, die aus Mischungen von Polyurethanlösungen mit wäßrigen Polyurethandispersionen (siehe z.B. Angew. Makromol. Chem. 98 (1981) 133 und die schon erwähnte DE-OS 2427 274 und die dort zitierten Druckschriften) bestehen. Die Polyurethandispersionen

können nichtionisch oder vorzugsweise ionisch sein, wobei die ionischen Reste z.B. $-SO_3^-$, $-COO^-$ oder $-N^+R_3$-Gruppen sein können.

Ionische Polymersysteme sind besonders bevorzugt, da sie eine gute Haftung zur Trägerfolie und eine Immobilisierung der für die Nachweisreaktion erforderlichen Enzyme zeigen. Die erfindungsgemäßen Nachweissysteme auf Basis ionischer Polymerer können mehrere Stunden lang mit Wasser gespült werden, ohne daß nennenswertes Ausbluten eintritt.

Zum Herstellen der Gießlösungen werden die für die jeweiligen Polymeren gängigen Lösungsmittel, wie z.B. Dimethylformamid, N-Methylpyrrolidon oder Dioxolan verwendet, wobei gegebenenfalls anorganische, in der Gießlösung lösliche Salze, wie LiCl, $CaCl_2$ oder $Mg(ClO_4)_2$ als Porenbildner zugesetzt werden können. Als weitere Additive können auch Stoffe, die in der Gießlösung unlöslich sind und als Füllstoffe dienen, wie $SiO_2$, $TiO_2$ (vgl. Europ. Patentanmeldung 0 077 509), $BaSO_4$, ZnO oder Cellulose- bzw. Agarosepulver, an denen gegebenenfalls biologisch aktives Material immobilisiert ist, zugesetzt werden.

Die Herstellung erfindungsgemäßer, trägergestützter, mikroporöser Nachweiselemente erfolgt dadurch, daß ein geeigneter Träger mit einer derartigen Gießlösung gleichmäßig beschichtet (Schichtdicke ca. 50 - 100 µm) und vorzugsweise gleich anschließend in ein Koagulationsbad getaucht wird, wobei die mikroporösen, trägergestützten Polymerfilme entstehen. Die Zeit zwischen Beschichtung des Trägers und der Koagulation wird möglichst gering gehalten (ca. einige Sekunden) damit der Träger vom Lösungsmittel der Gießlösung nicht angegriffen wird. Als Koagulationsflüssigkeit eignen sich z.B. Wasser oder wäßrige Pufferlösungen, die gegebenenfalls auch Weichmacher, z.B. Glycerin, enthalten können.

Durch die Natur des Koagulationsbades läßt sich in einfacher Weise die Porenstruktur modifizieren. So können aus nur einer Gießlösung durch Fällen in Wasser bei steigenden Temperaturen zunehmende Porengrößen an der Membranoberfläche erzielt werden, wie sich durch REM-Aufnahmen von mikroporösen Polymerfilmen, die bei Raumtemperatur , 45° C und 60° C koaguliert wurden, zeigen läßt. Ähnliche Effekte lassen sich auch durch Verwendung von Gemischen aus Wasser mit organischen Lösungsmitteln, z.B. Wasser-Dimethylformamid erzielen.

Als Träger für die erfindungsgemäßen Nachweiselemente eignen sich prinzipiell makroskopisch glatte Polymerfolien, auf denen das verwendete Polymersystem während der Koagulation und auch nach dem Trocknen eine gute Haftung zeigt und die durch die Polymergießlösung keine Veränderung erleiden. Besonders bevorzugt werden transparente Polymerfolien, die auch durch die Trägerseite eine Auswertung der Farbreaktion ermöglichen. Ganz besonders bevorzugt werden transparente Polyethylenterephthalatfolien.

Die für die Nachweisreaktion erforderlichen Reagenzien können auf verschiedene Art in die mikroporösen Polymerfilme gebracht werden, beispielsweise durch Einrühren-in die Gießlösung, durch nachträgliches Tränken der porösen Filme oder durch eine Kombination dieser beiden Verfahren.

In die erfindungsgemäßen Testvorrichtungen werden an sich bekannte enzymhaltige Reagenzsysteme für den Nachweis eines Analyten eingebracht.

Eine einfache Methode zur Herstellung von mit Nachweisreagenzien beladenen mikroporösen Polymerfilmen besteht z.B. darin, daß man das verwendete Chromogen (z.B. 3,3',5,5'-Tetramethylbenzidin) in der Gießlösung löst und diese nach dem Koagulationsverfahren zu einem chromogenbeladenen, mikroporösen Film verarbeitet, der dann mit dem (gegebenenfalls gepufferten) Enzymsystem getränkt wird.

Die erfindungsgemäßen Testmittel können auch als Mehrschichtensystem ausgebildet sein. Das ist dann von Vorteil, wenn man auf eine Trennung der Nachweisreagenzien angewiesen ist. Beispielsweise kann eine Trägerfolie mit einer Polymerschicht versehen werden, auf die dann die asymmetrische Membran nach der beschriebenen Koagulationsmethode aufgebracht, oder eine separat hergestellte, trägerfreie, fertige Membran auflaminiert wird.

In der zwischen Träger und Membran befindlichen Polymerschicht können gegebenenfalls alle oder ein Teil der Nachweisreagenzien eingearbeitet sein, wobei sich der andere Teil der Nachweisreagenzien in der darüber befindlichen Membran befindet.

Als polymere Zwischenschicht, in die gegebenenfalls Nachweisreagenzien eingearbeitet sein können (Reagenzienschicht), eignen sich z.B. an sich bekannte Filme aus wäßrigen Dispersionen, die zur Klasse der Polyvinylverbindungen, der Vinylmischpolymerisate, der Polystyrolsulfonsäuren, der Polyamide oder der Polyurethane gehören. Da die reagenzienhaltige Schicht vorzugsweise in Wasser löslich oder mindestens quellbar sein soll, sind Polyurethandispersionen, die gegebenenfalls mit wasserlöslichen oder wasserquellbaren Polymeren, wie Polyvinylalkohol, Polyethylenglykol, Celluloseether, Polyacrylamid, Polyacrylsäure oder Polyvinylpyrrolidon gemischt werden, besonders geeignet. Ganz besonders bevorzugte Reagenzschichten werden aus Mischungen ionischer Polyurethandispersionen mit Polyvinylpyrrolidon erhalten.

Das Beobachten der Farbreaktion kann, nach Abwischen des Probenüberschusses, von der Aufgabenseite (Membranoberfläche) her, oder bei Verwendung von transparenten Trägern auch von der Trägerseite

her erfolgen, wobei der Probenüberschuß nicht abgewischt zu werden braucht. Im letzteren Fall ist es bevorzugt, wenn sich das Chromogen in einer Reagenzschicht zwischen Membran und transparentem Träger befindet.

Die erfindungsgemäßen Nachweiselemente eignen sich für die quantitative Bestimmung von nieder- und hochmolekularen Komponenten in flüssigen Proben, insbesondere für den quantitativen spektrophotometrischen Nachweis von Inhaltsstoffen in Körperflüssigkeiten, wie z.B. Bilirubin, Ketone, Triglyceride, Harnstoff oder Haemoglobin. Ganz besonders geeignet sind die erfindungsgemäßen Nachweissysteme, wie die anschließenden Beispiele zeigen, für den quantitativen Glucose-Nachweis im Vollblut, sowie für den Nachweis von Enzymen wie Glucoseoxidase oder Cholinesterase, den Nachweis von Bilirubin oder Keton-Körpern.

Mengenangaben in den Beispielen sind, wenn nicht anders vermerkt, als Gewichtsteile bzw. Gewichtsprozente zu verstehen.

Beispiel 1

Glucose-Nachweis

Mit Hilfe eines schnelldrehenden Rührers (Dissolvers) wurde eine Gießlösung folgender Zusammensetzung hergestellt:

8.02 g Disulfimid-Polyamid
2.40 g $CaCl_2$
43.02 g Dimethylformamid (DMF)
0,01 g Ascorbinsäure
0,01 g Natriumcitrat
0.40 g Citronensäure
0.48 g 3,3',5,5'-Tetramethylbenzidin
45.40 g Titandioxid
0.13 g Peroxidase (POD, 277 U/mg)
0.13 g Glucoseoxidase (GOD, 116 U/mg)

Mit dieser Gießlösung wurde eine Polyethylenterephthalatfolie mit Hilfe eines Rakelmessers in einer Ziehstärke von 100 µm gleichmäßig beschichtet. Dieser trägergestützte Film wurde 10 Min. in einem 30 %igen wäßrigen Glycerinbad koaguliert. Die dabei entstandene feste, trägergestützte Membran wurde mit warmer Luft (35 °C) getrocknet und mit Vollblut auf ihre Funktionsfähigkeit überprüft. Das Blut wurde auf die Membranoberfläche gegeben und nach einer Minute abgewischt. Etwa 30 Sek. nach dem Abwischen war auf der Membranoberfläche eine homogene Grünfärbung entstanden.

Bei dem Disulfimid-Polyamid handelt es sich um ein in einer Eintopfreaktion gemäß US-PS 4 269 967 hergestelltes Polykondensat aus:

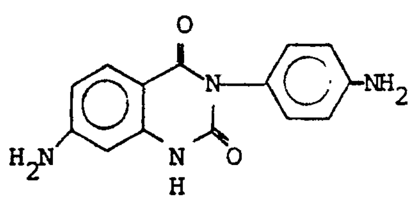
+
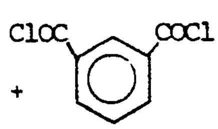
+
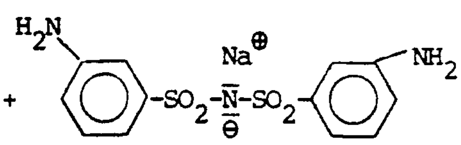

Beispiel 2

Glucose-Nachweis

Gießlösung: 13.73 g Polyurethan 66.37 g Dimethylformamid 7.24 g Polyurethandispersion in Wasser/DMF 0.07 g Natriumdioctylsulfosuccinat 11.01 g Titandioxid 0.79 g 3,3',5,5'-Tetramethylbenzidin 0.79 g Ascorbinsäure.

Bei dem verwendeten Polyurethan handelt es sich um ein thermoplastisches Material, welches durch Umsetzung von 75 Teilen eines Polyesters aus Adipinsäure, 70 Mol-% Ethylenglykol und 30 Mol % 1,4-Butandiol (MG = 2000),

25 Teilen eines Polyesters aus Adipinsäure und 1,4-Butandiol (MG = 2250),
25 Teilen 1,4-Butandiol und
85 Teilen Diphenylmethandiisocyanat
erhalten wurde.

Die Polyurethandispersion dient als Koagulierhilfsmittel und ist eine kationische, emulgatorfreie Dispersion eines Umsetzungsproduktes aus
200 Teilen eines Polyesters aus Adipinsäure, Phthalsäure und Ethylenglykol (MG = 1700),
50 Teilen Toluylendiisocyanat,
20 Teilen N-Methyldiethanolamin und
6 Teilen p-Xylylendichlorid.

Die Herstellung der trägergestützten, mikroporösen Polymermembran erfolgte wie in Beispiel 1 mit folgenden Veränderungen:
Träger: Polyethylenterephthalatfolie
Koagulationsbad: 1 %ige Lösung von Na-Laurylsulfat.

Nach dem Trocknen wurde der Film 1 Min. in einer 1 %igen POD (277 U/mg)/GOD (116 U/mg)-Lösung in Citratpuffer (pH 5,5) getränkt und getrocknet.

Test mit Vollblut: 10 Sek. nach der Probenaufgabe war durch den transparenten Träger eine homogene Blaufärbung zu beobachten, ebenso nach Abwischen des Probenüberschusses an der Membranoberfläche.

Mit 0,05, 0,1 und 0,5 %igen Glucoselösungen ergaben sich sofort homogene Blaufärbungen, die entsprechend der steigenden Glucosekonzentration zunehmende Farbintensitäten (Farbabstufungen) zeigten.

Dementsprechend konnten bei den reflektometrischen Auswertungen für verschiedene Glucosekonzentrationen abgestufte Reflektionswerte gemessen werden. Außerdem zeigten die Messungen, daß die Verfärbung in Abhängigkeit von der Glucosekonzentration im Bereich von 20 bis 800 mg Glucose/dl Wasser linear ist und daß der Endpunkt der Nachweisreaktion nach spätestens 40 Sekunden erreicht ist. Dies ist im Vergleich zu bekannten Testvorrichtungen als außerordentlich schnell anzusehen.

Die elektronenmiskroskopischen Untersuchungen (REM) des in Beispiel 2 beschriebenen Teststreifensystems zeigten, daß es sich um eine hochporöse Membran handelt, wobei die mittlere Porengröße 0,5 µ beträgt.

Beispiel 3

Glucose-Nachweis

Zweischichtensystem

A) Herstellen einer trägergestützten Reagenzienschicht
8.40 g wäßrige Polyurethandispersion
3.00 g Polyvinylpyrrolidon (MG 350000)
0.50 g Tetramethylbenzidin (gelöst in 1 g Ethylacetat)
je 0.12 g Glucoseoxidase (116 U/mg) und Peroxidase (277 U/mg)
und 0.025 g Ascorbinsäure
werden zusammengerührt, auf eine Polyesterfolie beschichtet und mit Warmluft getrocknet, wobei man eine trägergestützte Reagenzschicht erhält.
Bei der Polyurethandispersion handelt es sich um eine 40 %ige wäßrige Dispersion eines Umsetzungsproduktes aus
82 Teilen eines Polyesters aus Adipinsäure, Hexandiol und Neopentylglykol (MG = 1700),
15 Teilen Hexamethylendiisocyanat,
2 Teilen Na-Ethylendiamin-ethanolsulfonat und
1 Teil Ethylendiamin.
Diese trägergestützten Reagenzschichten ergaben mit wäßrigen Glucoselösungen ca. 20 Sek. nach der Probenaufgabe homogene, mit unterschiedlichen Glucosekonzentrationen abgestufte Verfärbungen (vgl. Beispiel 2).
B) Aufbringen einer asymmetrischen Phaseninversionsmembran
a) alle Nachweisreagenzien in der Reagenzschicht
Auf die in Beispiel 3A) beschriebene trägergestützte Reagenzschicht wurde die in Beispiel 2 beschriebene Gießlösung, die jedoch das dort beschriebene Tetramethylbenzidin sowie die Ascorbinsäure nicht enthielt, beschichtet und in 1 %iger wäßriger Na-Laurylsulfatlösung koaguliert.
Nach dem Trocknen mit warmer Luft wurde mit wäßrigen Glucoselösungen sowie Vollblut getestet. Es konnten ca. 30 Sekunden nach der Probenaufgabe von der Trägerseite aus homogene, konzentrationsabhängige Blaufärbungen beobachtet werden.
b) Reagenzien in verschiedenen Schichten
Aus

| | |
|---|---|
| 8.40 g | wäßriger Polyurethandispersion (siehe Beispiel 3) |
| 3.00 g | Polyvinylpyrrolidon (MG 350 000) und |
| je 0.12 g | Glucoseoxidase (116 U/mg) und Peroxidase (277 U/mg) |

wurde in Analogie zu Beispiel 3 A) eine trägergestützte, enzymhaltige Schicht hergestellt. Darauf wurde die in Beispiel 2 beschriebene Gießlösung beschichtet, in 1 %iger Na-Laurylsulfatlösung koaguliert und getrocknet. Beim Test mit Vollblut konnte ca. 40 Sek. nach Probenaufgabe und Abwischen der Erythrozyten eine homogene Blaufärbung von der Aufgabeseite aus beobachtet werden. Mit den unterschiedlich konzentrierten Glucoselösungen ergaben sich abgestufte Blaufärbungen.

Beispiel 4

Glucose-Nachweis

Zweischichtensystem mit Reagenzschicht.

Gießlösung für die Reagenzschicht:
8.00 g der Polyurethandispersion aus Beispiel 3
1.00 g Polyvinylpyrrolidon (MG 10 000)
0.05 g Tetramethylbenzidin, gelöst in 10 g Ethylacetat
0.10 g Glucoseoxidase (116 U/mg)
0.10 g Peroxidase (277 U/mg) gelöst in 2 ml Wasser
0.01 ml einer 5 %igen wäßrigen Ascorbinsäurelösung
wurden zusammengerührt, auf eine Polyethylenterephthalatfolie beschichtet und mit Warmluft getrocknet (= trägergestützte Reagenzschicht).

Auf diese trägergestützte Reagenzschicht brachte man eine nach dem Phaseninversionsverfahren hergestellte Polyamidmembran aus folgender Gießlösung:

| | |
|---|---|
| 8.10 g | Polyamid (Polykondensationsprodukt aus Hexamethylendiamin und Isophthalsäure gemäß DE-OS 27 43 515) |
| 2.40 g | $CaCl_2$ |
| 43.00 g | Dimethylformamid |
| 46.00 g | $TiO_2$ |

Beschichtungsstärke: 100 µm
Koagulationsbad: 30 %ige wäßrige Glycerinlösung

Test mit Vollblut: Auf die Membranoberfläche wurde ein Tropfen Blut gegeben. Etwa 10 Sek. nach der Probenaufgabe konnte durch den transparenten Träger eine homogene Blaufärbung beobachtet werden.
Mit unterschiedlich konzentrierten Glucoselösungen (vgl. Bsp. 2) ergaben sich abgestufte Blaufärbungen.

Beispiel 5

Enzym-Nachweis

Die in Beispiel 2 beschriebene Membran wurde nach dem Trocknen mit einer 1 %igen wäßrigen Glucose/POD (277 U/mg>-Lösung getränkt und getrocknet.

Beim Test mit verdünnten GOD-Lösungen (20 U/ml; 40 U/ml; 80 U/ml; 120 U/ml; 160 U/ml) war sofort nach der Probenaufgabe an der Membranoberfläche sowie durch den transparenten Träger eine homogene Blaufärbung zu beobachten, die entsprechend der GOD-Konzentration abgestuft war.

Beispiel 5a

Cholinesterase-Teststreifen

Gießlösung und Herstellung der Membran wie in Beispiel 2, jedoch ohne TMB und Ascorbinsäure.

Die Membran wurde in einer Lösung aus 40 mg Indoxylacetat in 5 ml Ethylacetat und 120 mg 2-Methoxy-4-morpholino-benzoldiazoniumchlorid . $ZnCl_2$ in 5 ml Methanol und anschließend nochmals mit Tris-HCl-Puffer (0,4 M; pH 7,5) getränkt und getrocknet und dann zu Teststreifen verarbeitet.

Die Teststreifen verfärben sich nach Aufgabe von Cholinesteraselösungen und Serum je nach Enzymaktivität unterschiedlich schnell blau. Die Verfärbung kann mit einem Reflektionsmessgerät quantitativ ausgewertet werden.

## Patentansprüche

1. Testvorrichtung für den reflektrometrischen Nachweis einer Komponente in einem wässrigen Testmedium wobei die Vorrichtung eine Trägerschicht, eine mikroporöse Polymerschicht, gegebenenfalls weitere Schichten sowie inkorporiert in einer oder mehreren der Schichten Reagentien für den Nachweis der zu bestimmenden Komponente umfaßt, dadurch gekennzeichnet, daß die mikroporöse Polymerschicht eine nach dem Koagulationsverfahren hergestellte Membran mit asymmetrischer Porenstruktur ist, wobei sich die Poren zur Seite der Probenaufgabe hin verengen und das Polymer ausgewählt ist aus der Gruppe Polyamide, Polyamide mit Disulfimidgruppen, Polyethercarbonate, Polyacrylnitril und Polyurethane und daß die Trägerschicht makroskopisch glatt und vorzugsweise unter den Testbedingungen für die Probe undurchlässig ist und die Reagentien enzymhaltig sind.

2. Testvorrichtung nach Anspruch 1 worin das Verhältnis der mittleren Porendurchmesser an der Membranunterseite zu jenen an der Membranoberfläche großer als 5:1 ist.

3. Testvorrichtung nach den Ansprüchen 1 und 2 zusätzlich enthaltend Polymere die ionische Gruppen aufweisen und/oder unlösliche Füllstoffe.

4. Testvorrichtung nach Anspruch 3 wobei die ionischen Polymere ausgewählt sind aus der Gruppe Polyvinylverbindungen, Vinylmischpolymerisaten, Polystyrolsulfonsäuren, Polymaide und Polyurethane.

5. Testvorrichtung nach den Ansprüchen 3 und 4, wobei die ionischen Reste aus der Gruppe quartäres Amin, $-SO_3^-$ und $-COO^-$ stammt.

6. Testvorrichtung nach den Ansprüchen 3 bis 5, wobei der unlösliche Füllstoff aus der Gruppe $SiO_2$, $TiO_2$, $BaSO_4$, ZnO, Cellulose oder Agarose stammt.

7. Analytische Methode zum Nachweis einer Komponente in einem wässrigen Testmedium, dadurch gekennzeichnet, daß man eine Testvorrichtung gemäß Anspruch 1 bis 6 mit der Probe in Kontakt bringt, wobei die Probenaufgabe in der Weise erfolgt, daß die Probe in die Membran an der Oberfläche mit dem engeren Porendurchmesser eintritt und die Nachweisreaktion verfolgt.

8. Methode nach Anspruch 7, dadurch gekennzeichnet, daß man die Probe auf die Membranoberfläche aufbringt und die Nachweisreaktion von der Aufgabenseite her beobachtet.

9. Methode nach den Ansprüchen 7 und 8, dadurch gekennzeichnet, daß man eine Testvorrichtung mit transparenter Trägerschicht verwendet und die Nachweisreaktion von der Trägerseite her beobachtet.

10. Methode nach den Ansprüchen 7 bis 9, wobei die flüssige Probe eine Körperflüssigkeit wie Harn, Serum oder Blut ist.

## Claims

1. Test device for the reflectometric detection of a component in an aqueous test medium, the device comprising a support layer, a microporous polymer layer, where appropriate other layers and, incorporated in one or more of the layers, reagents for the detection of the component to be determined, characterised in that the microporous polymer layer is a membrane which has an asymmetric pore structure and is produced by the coagulation process, the pores narrowing towards the side to which the sample is applied and the polymer being selected from the group comprising polyamides, polyamides with disulphimide groups, polyether carbonates, polyacrylonitrile and

polyurethanes, and in that the support layer is macroscopically smooth and preferably impermeable to the sample under the test conditions, and the reagents contain enzymes.

2. Test device according to Claim 1, wherein the ratio of the mean pore diameter on the underside of the membrane to that on the membrane surface is greater than 5:1.

3. Test device according to Claims 1 and 2 additionally containing polymers which have ionic groups, and/or insoluble fillers.

4. Test device according to Claim 3, where the ionic polymers are selected from the group comprising polyvinyl compounds, vinyl copolymers, polystyrenesulphonic acids, polyamides and polyurethanes.

5. Test device according to Claims 3 and 4, where the ionic radicals are derived from the group comprising quaternary amine, $-SO_3^-$ and $-COO^-$.

6. Test device according to Claims 3 to 5, where the insoluble filler is derived from the group comprising $SiO_2$, $TiO_2$, $BaSO_4$, ZnO, cellulose or agarose.

7. Analytical method for the detection of a component in an aqueous test medium, characterised in that a test device according to Claim 1 to 6 is brought into contact with the sample, the sample being applied in a manner such that the sample enters the membrane at the surface having the narrower pore diameter, and the detection reaction is followed.

8. Method according to Claim 7, characterised in that the sample is applied to the membrane surface, and the detection reaction is observed from the application side.

9. Method according to Claims 7 and 8, characterised in that a test device having a transparent support layer is used, and the detection reaction is observed from the support side.

10. Method according to Claims 7 to 9, where the liquid sample is a body fluid such as urine, serum or blood.

**Revendications**

1. Dispositif d'essai destiné à l'identification par réflectométrie d'un composant dans un milieu d'essai aqueux, le dispositif comprenant une couche support, une couche de polymère microporeuse, le cas échéant d'autres couches, ainsi que des réactifs incorporés dans une ou plusieurs des couches, en vue de l'identification du composant à déterminer, caractérisé en ce que la couche de polymère microporeuse est une membrane à pores de structure asymétrique, fabriquée selon le procédé de la coagulation, les pores se rétrécissant en direction de la face où est déposé l'échantillon et le polymère étant sélectionné dans le groupe des polyamides, polyamides à groupements disulfimide, polyéthercarbonates, polyacrylonitrile et polyuréthannes et en ce que la couche support est macroscopiquement lisse et de préférence imperméable à l'échantillon dans les conditions de l'essai et que les réactifs sont enzymatiques.

2. Dispositif d'essai selon la revendication 1, dans lequel le rapport entre le diamètre moyen des pores sur la face inférieure de la membrane et à la surface de la membrane est supérieur à 5 : 1.

3. Dispositif d'essai selon les revendications 1 et 2, contenant en outre des polymères présentant des groupements ioniques et/ou des charges insolubles.

4. Dispositif d'essai selon la revendication 3, les polymères ioniques étant sélectionnés dans le groupe des composés polyvinyliques, des copolymères vinyliques, des acides polystyrènesulfoniques, des polyamides et des polyuréthannes.

5. Dispositif d'essai selon les revendications 3 et 4, les radicaux ioniques provenant du groupe amine quaternaire, $-SO_3^-$ et $-COO^-$.

6. Dispositif d'essai selon les revendications 3 à 5, la charge insoluble provenant du groupe $SiO_2$, $TiO_2$, $BaSO_4$, ZnO, cellulose ou agarose.

7. Procédé analytique d'identification d'un composant dans un milieu d'essai aqueux, caractérisé en ce qu'on met un dispositif d'essai selon les revendications 1 à 6 en contact avec l'échantillon, le dépôt de l'échantillon s'effectuant de façon telle que l'échantillon pénètre dans la membrane par la surface présentant le diamètre de pores le plus fin, et qu'on suit la réaction d'identification.

8. Procédé selon la revendication 7, caractérisé en ce qu'on dépose l'échantillon à la surface de la membrane et qu'on observe la réaction d'identification à partir de la face où est déposé l'échantillon.

9. Procédé selon les revendications 7 et 8, caractérisé en ce qu'on utilise un dispositif d'essai à couche support transparente et qu'on observe la réaction d'identification à partir de la face support.

10. Procédé selon les revendications 7 à 9, l'échantillon liquide étant un liquide corporel tel que l'urée, le sérum ou le sang.